# EUROPEAN PATENT APPLICATION

(11) **EP 0 747 002 A1**
(43) Date of publication of application: **11.12.1996**
(21) Application number: 96303000.2
(22) Date of filing: 29.04.1996
(51) Int. Cl.: A61B 5/00, A61B 5/103, G01N 33/72

(54) **Non-invasive bilirubin monitor**

(30) Priority: 06.06.1995 US 469330
(71) Applicant: OHMEDA INC., Liberty Corner, New Jersey 07938-0804 (US)
(72) Inventor: Buttitta, Anthony D., Libertyville, Illinois 60048 (US); Falk, Steven M., Silver Spring, Maryland 20901 (US)
(74) Representative: Gough, Peter

(57) **Abstract**

A bilirubin monitor that utilises two wavelengths of light radiation directed to an infants arterial system. The monitor includes a light detector that receives light preferably reflected or backscattered from that arterial system and signal processing circuits compute a ratio based upon the peak to peak values of the AC component of the signals from the light detector with respect to each of the wavelengths. One wavelength is absorbed by the bilirubin in the infant's blood while the other wavelength radiation is either not affected by the bilirubin concentration or is absorbed substantially less than the first wavelength.

## Description

The present invention relates to the a non-invasive monitor for the measurement of the level of bilirubin in the bloodstream of an infant, and, more particularly, to the use of certain light sources of predetermined wavelengths to be directed on to the infant's skin to penetrate to the infant's arterial bloodstream. The reflectivity or backscatter of the light from the infant's bloodstream from the light sources is detected and measured to determine the bilirubin level of that arterial system of the infant.

Hyperbilirubinemia is an affliction of new-born infants typified by an elevated level of a toxic molecule known as bilirubin in the infant's blood. Current medical therapy for such an affliction is through the use of phototherapy where light radiation, generally within certain desired wavelengths, is directed upon the infant's skin.

In general, such light therapy is administered by banks of florescent lights positioned above the infant to direct the radiation downwardly upon the infant's skin. However, more recently, fiberoptic woven pads have been used which receive the proper wavelength radiation from a light source and which are placed near the infant. Radiation from the fiberoptic pad is emitted outwardly to impinge upon the infant.

In either instance, it is advantageous for the operator to know, on a fairly current basis, the level of bilirubin in the infant's blood so that the operator knows how much light therapy to administer, either with respect to the intensity of the light radiation and/or the length of time the therapy needs to be administered.

Accordingly, a monitor is desired to ascertain continuously the infant's bilirubin level to keep the operator currently apprised of the infant's status.

There are three major techniques for measuring the level of bilirubin in an infant's blood. The first is by measuring the yellow colour of the infant's skin visually, that is, the operator judges the level of bilirubin by visually perceiving the skin and subjectively making a determination. Obviously, while the method may be adequate for a gross screening procedure, it does not actually make a measurement of the bilirubin and is not precise enough to guide an operator in the amount of light therapy to administer.

The second method is the most frequently used and involves the actual drawing of blood from the infant for a serum bilirubin analysis by chemical or spectrophotometric means. While this method is certainly more accurate, its major drawback is that the blood samples need to be drawn from the infant several times a day. The most popular method of withdrawing blood from the infant is by means of a "heel stick" where a needle is introduced into the infant's heel to withdraw the blood.

This method causes considerable trauma to the infant in the form of emotional trauma as well as pain. Further the emotional trauma to the parents having to administer a heel stick to their own infant decreases the feasibility of the method being used in the home. Additionally, it is not a continuous monitor since a sample of blood needs to be taken for each reading and the sample transferred to an instrument for making the evaluation.

Finally, there are non-invasive techniques currently used, one of which is shown and described in U.S. Patent 4,267,844 of Minolta Inc. and which utilises flashing white light into the infant, collecting the blue and green component of the back scattered light, and using the backscattered intensities at the selected wavelengths to calculate the level of bilirubin in the infant's blood.

The aforesaid Minolta technique, however, measures transcutaneous bilirubin (TcB) and in doing so, incurs some inherent inaccuracies. In particular, the inaccuracies are caused by intermeter, intrameter, interoperator, intraoperator and intrasubject variabilities, as well as bias from skin pigmentation, carotene, skin thickness and other blue-green absorbing components. In addition, since the system measures TcB and not blood serum bilirubin, the results are artificially influenced by the phototherapy light source itself. Thus where phototherapy is being applied, the more accurate method requires a serum bilirubin analyses, and which gets back to the second method and the trauma associated therewith.

Accordingly, the need for a non-invasive, relatively rapid monitor to determine the level of bilirubin in an infant is needed having sufficient accuracy and which is easy to use, relatively independent of outside influences and which is of sufficient reliability.

It is an aim of the present invention to provide a system and method which can be utilised to obtain rapidly and non-invasively a measurement of the concentration of bilirubin in the infant patients blood.

According to one aspect of the present invention, a system for determining the bilirubin concentration within the arterial blood of a patient, is characterised by:
(a) a first light source for producing light of a known first wavelength that is absorbed by bilirubin in the arterial blood of the patient;
(b) a second light source for producing light of a known second wavelength that is absorbed by the bilirubin in the arterial blood at a rate significantly less than the absorption rate of the first light source;
(c) means to position said first and second light sources to direct the light produced therefrom onto the skin of the patient such that the light radiation reaches the arterial system of the patient;
(d) a light detector means located adjacent the skin of the patient for receiving light attenuated by absorption in the patients arterial system as a result of the light from said first and second light sources and producing electrical signals representative of such attenuated light; and
(e) signal processing means for receiving the signals from said light detector means, said signal processing means producing an output signal representative of the bilirubin concentration in the arterial system of the patient.

According to a further aspect of the present invention a method of determining the bilirubin concentration within the arterial blood of an infant comprises the steps of:
(a) directing light of a first wavelength that is absorbed by bilirubin onto the infant to reach the infant's bloodstream;
(b) directing light of a second wavelength that is not absorbed by bilirubin onto the infant to reach the infant's bloodstream;
(c) detecting light attenuated by the infant's bloodstream resulting from the light of each of the first and second wavelengths and producing an electrical signal representative of the intensity of such attenuated light for each of such wavelengths; and
(d) processing the electrical signals produced by step (c) to determine the concentration of bilirubin in the bloodstream of the infant.

The system and method of the present invention utilise the reflectance of light transmitted through an infants skin through a part of the infant within which arterial blood is flowing and detecting the light reflected or backscattered from the arterial blood vessels of the infant. The light radiation is provided at two wavelengths, one of which is within the absorption range of bilirubin and therefore is affected by the concentration of the bilirubin in the patients blood stream. The other wavelength is not affected or absorbed by the bilirubin but both wavelengths are absorbed equally by the haemoglobin (both reduced and oxygenated).

Accordingly, the first wavelength is about 454 nanometers while the second wavelength is preferred to be about 540 nanometers. In each instance, the light radiation at high energy and at the selected wavelengths is preferably directed through the infant's blood stream and a detector receives and produces signals representative of the light reflected or backscattered from the patients arterial blood stream. As an alternative, the light radiation at the selected wavelengths could be transmitted through the infant's blood stream and the absorption measured rather that the reflectance from the blood stream.

The detector thus produces two signals, each having a relatively small AC component and a relatively large DC component. The DC component represents a non-varying components which consists of skin pigmentation, skin thickness, patient-device coupling and other blue-green light absorbing or reflecting parameters. The AC signal represents the arterial blood and varies temporally with the oscillations of the arterial blood with the cardiac cycle. The signals from the detector are then processed to produce a ratio of the peak to peak AC signals of the light reflected at the two different wavelengths and which is indicative of the percentage or concentration of bilirubin in the blood of the infant.

In the preferred signal processing, the signals from the light detector are processed through a resettable peak-hold circuit such that the values of the peak signals from the detector are determined and held. Those peak signals are then digitised and the digital values used to process the data. Those digitised signals are transmitted to a microprocessor where the further processing takes place with digital data. In the microprocessor, the digital signal representing the peaks of signals from the detector are formed into a waveforms represented by a sine wave for both of the LED wavelengths.

The peak to peak measurement is thus made using that digital data and the values of the peak to peak waveforms for each of the waves are divided, the value of the peak to peak signal representative of the bilirubin absorbing wavelength divided by the peak to peak value representative of the signal from the non-absorbing wavelength.

A ratio is then obtained by the simple division of the peak to peak values at each wavelength and the resultant value is directly proportional to the concentration of bilirubin in the arterial blood of the infant. That ratio is processed via a linear equation to arrive at the actual value for the percentage of bilirubin in the infant's blood and that value may be displayed either immediately or trended to show values over a finite period of time.

Thus, the present system and method can rapidly obtain and display a value for the bilirubin concentration of the infant's blood in order to determine the amount of light therapy to apply to the infant, yet the entire process in non-invasive and causes no discomfort to the patient. In addition, the procedure is of high accuracy and is not affected by the differences of travel of the light radiation, skin differences and the like since the use of a ratio eliminates most of the interfering components that would lead to such inaccuracies.

An embodiment of the invention will now be described, by way of example, reference being made to the Figures of the accompanying diagrammatic drawings in which:-
Figure 1 is a schematic view of a bilirubin monitoring system constructed in accordance with the present invention;
Figure 2 is a block diagram of the signal processing used with the present invention to determine the concentration of bilirubin;
Figure 3 is a representation of the waveforms typically obtained in the signal processing circuits used to carry out the invention; and
Figure 4 is a still further representation, in a waveform, of certain digital data used by the microprocessor in carrying out the present invention.

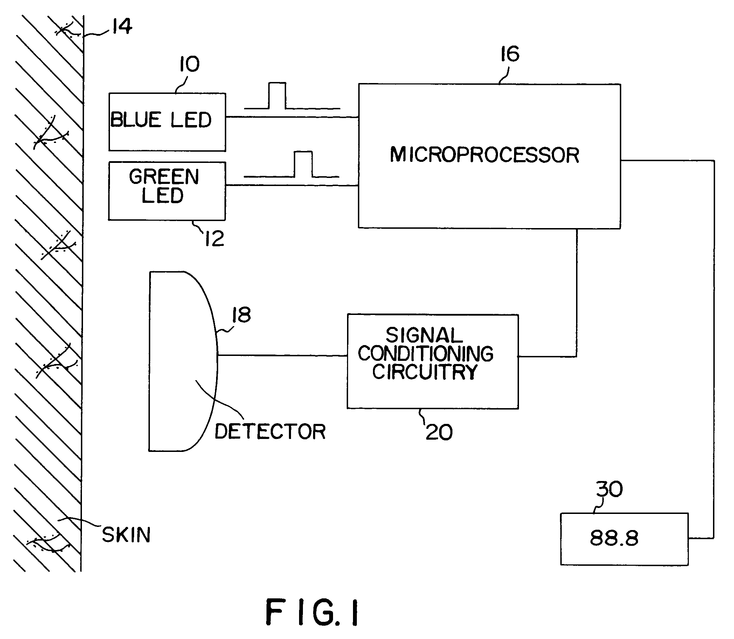

Turning first to FIG. 1, there is shown a schematic view of the bilirubin monitor of the present invention and showing a blue LED 10 and a green LED 12 directing the light radiation upon the skin 14 of an infant. As previously outlined, the blue LED 10 is of a wavelength that is absorbed by bilirubin and is preferably about 454 nanometers. That value is, of course, optimum and the actual wavelength may vary somewhat therefrom, and may be plus or minus 10 nanometers from the optimum value, i.e. from about 444 to 464 nanometers.

The green LED 12 is of a wavelength that is not absorbed by bilirubin and is unaffected by other pulsating components in the arterial bloodstream. The preferred wavelength of the green LED is about 540 nanometers, however, again, the actual wavelength may vary therefrom and may vary in the range of plus or minus about 40 nanometers from the 540 nanometer value, i.e. from about 500 nanometers to about 580 nanometers. As noted, the variance allowed with the green LED is much wider that the 454 nanometer wavelength which must be more accurate since the absorption in bilirubin is a relatively narrow band width. The importance of the choice of 540 nanometers is that it is absorbed in haemoglobin (both reduced and oxygenated) about the same extent as the 454 nanometer wavelength light is absorbed therein.

It should be noted that the 540 nanometer wavelength was selected since it is basically not absorbed by the bilirubin in the arterial blood, however, the wavelength chosen could be any wavelength that is absorbed by the bilirubin at a rate significantly less than the absorption rate of the 454 wavelength light radiation. The preferred wavelength for the less absorbed radiation is, of course, 540 nanometers where the radiation is not absorbed since the signals derived, as will be explained, are easier to process than where the light radiation is absorbed to some extent by the bilirubin, albeit significantly less that at the 454 nanometer wavelength.

As used herein, the sources of electromagnetic energy are described to be LED's as the most convenient and presently readily available, it being understood, however, that other sources of electromagnetic energy are suitable in carrying out the present invention.

As seen in Figure 1, therefore, the LED's 10 and 12 are driven by a conventional driver, preferably in the form of alternatingly timed pulsed waves that intermittently drive each of the LEDs at precise timed sequence, that is, the duty cycle is alternating LED activation times. The timed pulses used to activate the LED's 10 and 12 are preferably high energy since penetration of the light through the skin of the infant is difficult at the lower wavelengths in the blue to blue-green range. Accordingly considerable energy is needed for minute periods of time. The current needed may be in the range of about 10 amperes for 5 microseconds every 5 millisecond, that is, the LED's have a duty cycle of about .1%.

The timing can be controlled by microprocessor 16 and the timing sequence also used to alert the detection circuitry as to which LED is activated during the overall duty cycle.

A light detector 18, such as a photodiode, is positioned adjacent the LED's 10 and 12 along the infant's skin 14 and the light detector 18 receives the light reflected or backscattered from the arterial system of the infant such that the intensity of the light received by light detector 18 from each of the LED's has been attenuated by the absorption by the infant's arterial system. The light reflected and received by light detector 18 has also been attenuated by absorption of light from the green LED by bilirubin in the infants arterial system.

Accordingly, the light detector 18 generates signals corresponding to the light reflected from the infant's arterial system for both of the wavelengths of the LED's 10 and 12.

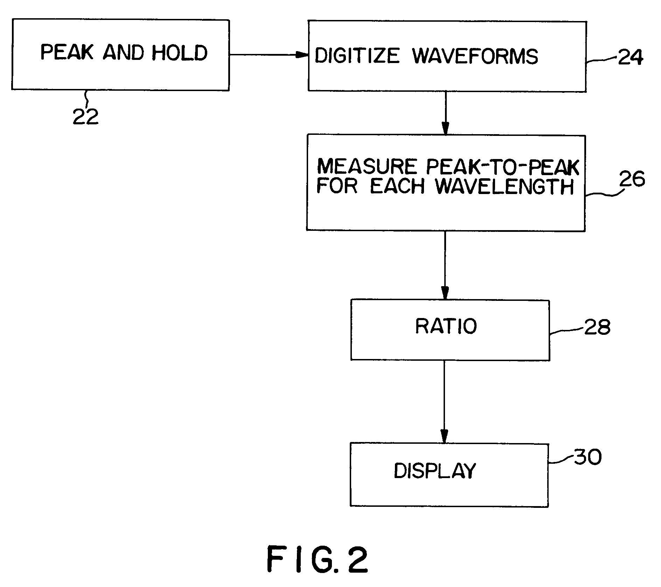

Turning now to FIG. 2, there is shown a block diagram of the signal processing preferably utilised to obtain a value of the bilirubin level of the infant from the backscattered light from the infant's arterial system. As noted, the signals from the light detector 18 are kept separate with respect to each of the LED's 10 and 12 and that signal train is fed into the Signal Conditioning Circuit 20 (FIG. 1) where the signals are processed in a Peak and Hold circuit 22 where the peaks of the waveforms from the light detector 18 are detected and that value held.

The values of the peak signals obtained by the Peak and Hold circuit 22 are then digitised by a digitizer 24 and those values transmitted to the microprocessor 16 where they are stored in a table to be used to determine the concentration of bilirubin in the infant. The digital conversion of the signals is readily carried out by a conventional A/D converter since the processor is merely holding the peak values of the signals from the Peak and Hold circuit 22. The information is sampled at a sufficiently fast rate to convert the signals to digital values with sufficient useful data and the sampling rate preferably is at least about 4 times the heart rate of the infant. A typical sampling rate may be in the order of about 20 to 25 hertz.

Accordingly, the peak to peak values are obtained from the digital data, depicted at block 26 in the microprocessor 16 for both of the LED's and the values of the peak to peak data determined and held.

By a division circuit 28, a ratio is then calculated of the AC signals, by dividing the peak to peak value of the 454 nm signal by the peak to peak value of the 540 nm signal. The resulting division calculation is directly proportional to the concentration of bilirubin in the patients blood and is converted to an actual value by a simple linear equation. Finally, that value is sent to a display 30 where the value is communicated to the personnel attending the infant.

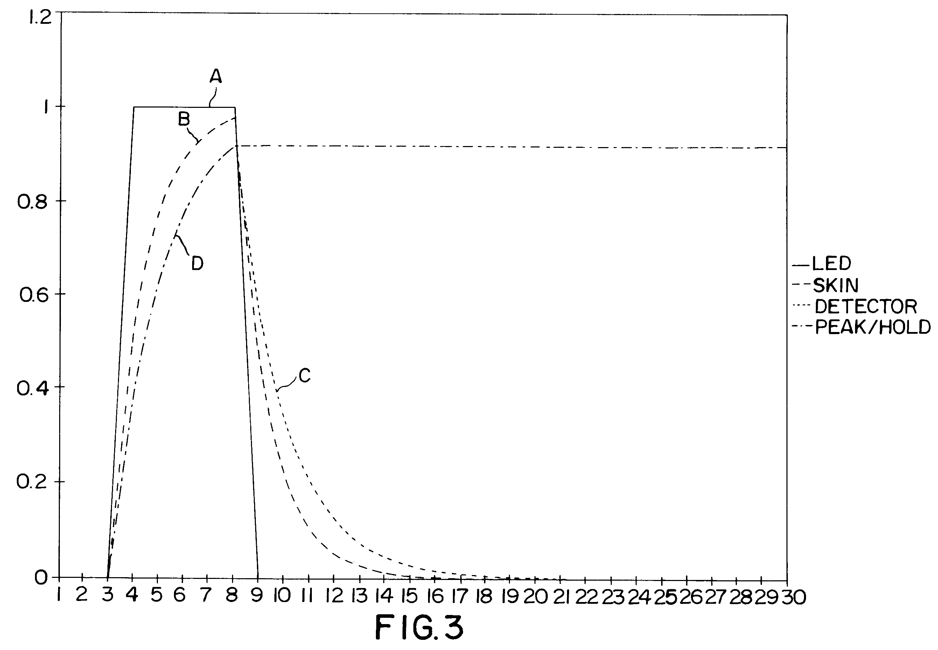

In Figure 3, there is shown the various waveforms generated and/or processed in carrying out the preferred method of signal processing. In all the waveforms of FIG. 3, although units are shown for the horizontal and vertical axies, the actual quantified units are not important to the description hereof and only the shapes of the waveforms are used to illustrate the signals that are present in carrying out the present invention. FIG. 3 is not intended to indicate any particular values for those waveforms.

Also, as is obvious, FIG. 3 represents the waveforms of one of the LED's and detectors, it being understood that a similar set of curves is applicable for both of the LED's of a similar nature, however one of which has the additional information resulting from the absorption of certain of the light in the 454 nanometer range by the bilirubin in the arterial blood of the infant.

As shown in FIG. 3, therefore, curve A is representative of the driving signal to the LED's and is a pulse that activates each of the LED's on an individual timed basis to turn each on and off. The light radiation backscattered from the infant's skin is depicted as curve B and, as shown that curve falls off rapidly as the light detector 18 and the typical loss can be seen in the output signal from the light backscattered from the infant.

Finally, in FIG. 3, curve D represents the output from the Peak and Hold circuit 22 and which basically detects the peak signal from the light detector 18 and holds that signal a specific value for use in the later signal processing. The Peak and Hold circuit 22 is continually reset to obtain and hold that peak signal from the light detector 18.

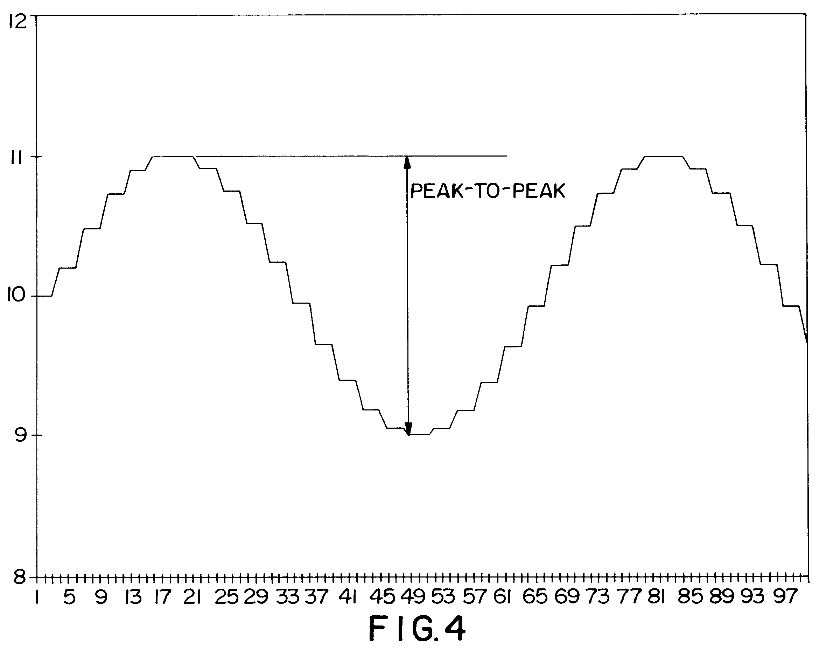

A curve representative of the digital data entering the microprocessor 16 is shown in Fig. 4 and is in the form roughly of a sine wave having a large DC offset and a very small AC component. Again, the FIG. 4 is typical of data representing the light radiation backscattered from both of the LED's 10 and 12 although only one wave form of the data is shown in the FIG. 4.

As previously stated, this data, in digital from is thus easily used to make a measurement of the peak to peak values for each of the LED's and a division circuit used to obtain a ratio of those peak to peak values. By a simple linear equation, that ratio is converted to a value representing the bilirubin level in the infant.

## Claims

1. A system for determining the bilirubin concentration within the arterial blood of a patient, said system characterised by:
(a) a first light source 10 for producing light of a known first wavelength that is absorbed by bilirubin in the arterial blood of the patient;
(b) a second light 12 source for producing light of a known second wavelength that is absorbed by the bilirubin in the arterial blood at a rate significantly less than the absorption rate of the first light source;
(c) means to position said first and second light sources (10,12) to direct the light produced therefrom onto the skin 14 of the patient such that the light radiation reaches the arterial system of the patient;
(d) a light detector means 18 located adjacent the skin 14 of the patient for receiving light attenuated by absorption in the patients arterial system as a result of the light from said first and second light sources 10, 12 and producing electrical signals representative of such attenuated light; and
(e) signal processing means 16 for receiving the signals from said light detector means 18, said signal processing means producing an output signal representative of the bilirubin concentration in the arterial system of the patient;

2. A system for determining bilirubin concentration as claimed in Claim 1 wherein the light received by said light detector 18 is backscattered from the patients arterial system.

3. A system for determining bilirubin concentration as claimed in Claim 1 or 2, wherein said light of said first predetermined wavelength is in the range of from about 444 to about 464 nanometers.

4. A system for determining bilirubin concentration in a patient as claimed in Claim 1, 2 or 3 wherein said light of said second predetermined wavelength is in the range of from about 500 to about 580 nanometers.

5. A system for determining bilirubin concentration in a patient as claimed in any one of Claims 1 to 4 wherein said light of said first predetermined wavelength is about 454 nanometers and the light of said second predetermined wavelength is about 540 nanometers.

6. A system for determining bilirubin concentration in a patient as claimed in any one of Claims 1 to 5 wherein said signals from said light detector 18 comprise pulses representative of the light received from each of said light sources 10, 12 and said signal processor 16 samples and holds the peak value of said pulses.

7. A system for determining bilirubin concentration in a patient as claimed in Claim 6 wherein said signal processor 16 digitises the pulses representing the peak values of said pulses from said light detector.

8. A system for determining bilirubin concentration in a patient as claimed in Claim 7 wherein said signal processing means 16 further comprises a microprocessor receiving the digital representations of said peak pulses and derives a peak to peak value for such pulses for light backscattered from each of said light sources.

9. A system for determining bilirubin concentration in a patient as claimed in Claim 8 wherein said microprocessor 16 comprises means to divide the peak to peak values to arrive at a ratio representative of the bilirubin concentration in the patients arterial blood stream.

10. A method of determining the bilirubin concentration within the arterial blood of an infant comprising the steps of:
(a) directing light of a first wavelength that is absorbed by bilirubin onto the infant to reach the infant's bloodstream;
(b) directing light of a second wavelength that is not absorbed by bilirubin onto the infant to reach the infant's bloodstream;
(c) detecting light attenuated by the infant's bloodstream resulting from the light of each of the first and second wavelengths and producing an electrical signal representative of the intensity of such attenuated light for each of such wavelengths; and
(d) processing the electrical signals produced by step (c) to determine the concentration of bilirubin in the bloodstream of the infant.
